# EUROPEAN PATENT APPLICATION

(11) **EP 4 806 463 A1**
(43) Date of publication of application: **16.09.2026**
(21) Application number: 25163675.9
(22) Date of filing: 13.03.2025
(51) Int. Cl.: A61K 41/00, A61K 47/69, A61P 9/00, B82Y 5/00

(54) **NANO-PARTICLE-BASED COMPOUND FOR MEDICAL TREATMENT**

(71) Applicant: Medizinische Hochschule Hannover, 30625 Hannover (DE); Gottfried Wilhelm Leibniz Universität Hannover, 30167 Hannover (DE)
(72) Inventor: Thum, Thomas, 30625 Hannover (DE); Hempel, Katharina, 30625 Hannover (DE); Kenneweg, Franziska, 30625 Hannover (DE); Kirschning, Andreas, 30167 Hannover (DE); Dräger, Gerald, 30167 Hannover (DE)
(74) Representative: Taruttis, Stefan Georg

(57) **Abstract**

A nano-particle-based compound for medical treatment for delivery of a pharmaceutical agent, the compound comprising a nanoparticle coupled to a pharmaceutical agent by a linker. The linker is covalently bound to the nanoparticle and covalently bound to the pharmaceutical agent. The compound is set up for controlled release of the pharmaceutical agent from the linker, wherein the release is controlled by heating of the nanoparticle, e.g. heating induced by alternating electric and/or magnetic fields. The linker has the advantage of allowing the release of the pharmaceutical agent without atoms of the linker remaining bound to the pharmaceutical agent.

## Description

### Nano-particle-based compound for medical treatment

The present invention provides a compound for use in medical treatment, especially for delivery of a pharmaceutical agent bound to a nano-particle, the nano-particle-based compound comprising or consisting of a nanoparticle coupled to a pharmaceutical agent by a linker. The linker is covalently bound to the nanoparticle and covalently bound to the pharmaceutical agent. The compound is set up for controlled release of the pharmaceutical agent from the linker, wherein the release is controlled by heating of the nanoparticle, e.g. heating induced by alternating electric and/or magnetic fields. The linker preferably has the advantage of allowing the release of the pharmaceutical agent without atoms of the linker remaining bound to the pharmaceutical agent. Preferably, the nanoparticle is paramagnetic or superparamagnetic.

### State of the art

Knipp et al., Tetrahedron 3422-3429 (2014), describes release of an amino compound or of a hydroxylated compound from esters, carbonates, carbamates and amides by intramolecular cyclization, concluding that thermal stability at 37 °C was only found for an amino carbonate motif leading to a seven-membered ring progenitor, higher temperatures releasing the hydroxylated compound.

Rezk et al., Materials & Design 234 (2023) 112350 describes magnetic nanoparticles coated with a thermoresponsive polymer with for release of doxorubicin upon heating the nanoparticles by an alternating magnetic field, for use in killing cancer cells by the drug release combined with heating. Doxorubicin release was ca. 29.5% at 37°C, 78.4% at 43°C.

### Object of the invention

It is an object of the invention to provide nanoparticles, preferably superparamagnetic nanoparticles, with a linker coupled to a pharmaceutical agent, wherein release of the pharmaceutical agent can be triggered by heating. Preferably, the pharmaceutical agent und shall be released without remnants of the linker remaining attached to the pharmaceutical agent. This is the case, if the pharmaceutical agent bears either an alcohol or an amino group. However, if such functional groups are missing, pharmaceutical agents can be slightly derivatized so that a hydroxyl group or an amino group becomes part of the pharmaceutical agent. In these cases a modified pharmaceutical agent is released which, however, has to retain its biological or medicinal properties, as e. g. in the case of locked RNA.

### Description of the invention

The invention achieves the object by the features of the claims, in particular by a compound comprising or consisting of a nano-particle coupled to a first end of a linker, the opposite second end of the linker terminating in a carbonyl group, which carbonyl group is bound to an amine group or a hydroxyl group of a pharmaceutical agent, wherein the linker carries the carbonyl group at the terminus of a linear hydrocarbon chain of 2, 3, 4 or 5 carbon atoms bound to a secondary amino group of the linker, optionally containing at least one heteroatom, e.g. oxygen forming an ether group in the hydrocarbon chain or sulfur forming a thioether group in the hydrocarbon chain. Optionally, the hydrocarbon chain of the linker can be substituted in at least one carbon atom by at least one group selected from C1- to C6 aliphatic groups, e.g. methyl, di-methyl, ethyl, di-ethyl, halogen, or a combination of two of these. The linker including its carbonyl group and its amino group preferably has a chain length of 5 or 6 carbon atoms, wherein one of the carbon atoms is optionally replaced by an oxygen or a sulfur atom, resulting in a chain length of 5 or 6 atoms of 4 or 5 carbon atoms plus one oxygen atom or plus one sulfur atom.It was found that two substituents to one carbon atom of the hydrocarbon chain of the linker supports the release of the pharmaceutical agent from the linker upon heating of the nanoparticle. Heteroatoms can be part of the chain, e. g. in the case of oxygen, the chain is an ether. Alternatively, at least two atoms of the linker, preferably formed by a linear hydrocarbon chain, can be derivatized by forming part of a ring system, e.g. at least two atoms of the linker forming part of an ortho-substituted (hetero)arene or of a 1,2-disubstituted aliphatic ring system, which is e,g. a 3-, a 4-, a 5- or a 6-membered ring. These aliphatic rings can be further modified with heteroatoms. Substituents to the arene or aliphatic ring can independently be selected from hydrogen, C1- to C6 aliphatic groups, e.g. methyl or di-methyl, and halogen. The secondary amino group of the linker is protected by an alkyl carbamate group, preferably by a tert-butoxycarbonyl group (Boc), or by another alkyl carbamate, e.g. methyl, ethyl or n-propyl or iso-propyl groups or C4 to C6 aliphatic linear or branched chains, a benzyl group, optionally derivatized in the benzene ring by at least one methoxy group. Opposite the linear hydrocarbon chain of 2, 3, 4 or 5 carbon atoms, optionally including in the chain at least one heteroatom, the first end of the linker is bound to the nano-particle via an amide group by an elongating portion, which elongating portion may have a chain length of at least 10 atoms, preferably at least 11 atoms, at least 12 atoms, at least 13 atoms, at least 14 or at least 15 atoms, e.g. up to 30 or up to 20 atoms. The elongating portion, which extends between the Boc-protected secondary amino group and the nano-particle, may be bound to the nano-particle directly or to at least one, e.g. two, three or four, in each case up to 100, e.g. up to 50, up to 20 or up to ten intermediate groups coupled to the nano-particle, each of which intermediate groups is e.g. a hexose or pentose, or poly- or oligomeric ethylenglycol or glycerol, and any combination of at least two of these, e.g. up to ten intermediate groups. Such intermediate groups that form the elongating portion between the first end of the linker and the nano-particle may e.g. be a dextran covering the nano-particle, or poly- or oligomeric ethylenglycol or glycerol.

Examples of the linker and of the elongating portion are shown for various combinations below. The exemplary linkers as well as the exemplary elongating portions can each independently be combined, e.g. any linker from the combinations shown can be combined with any elongating portion shown. Herein, the pharmaceutical compound is exemplified as LNA, coupled to the linker, the first end of which is bound to an elongating portion, the opposite end of which is bound to a nano-particle, preferably a super-paramagnetic nanoparticle, herein represented by a super-paramagnetic iron-oxide nanoparticle (SPION).

The linker which upon heating, e.g. heating above room temperature, preferably heating above body temperature (37-38°C in human patients) releases the pharmaceutical agent is depicted below, the linker extending from the carbonyl group to the tertiary, carbamate-protected amino group, with the carbonyl group bound to a pharmaceutical agent, preferably to an oxygen or nitrogen of the pharmaceutical agent. The pharmaceutical agent (represented below by LNA or other drugs) carries an oxygen or nitrogen, which after heating of the compound is released from the carbonyl of the linker, resulting in the pharmaceutical agent having a hydroxyl or amino group. The nanoparticle is bound to the carbamate-protected amino group of the linker, directly or by an elongating portion which connects the carbamate-protected amino group of the linker to the nanoparticle. Herein, the nanoparticle is exemplified by a SPION. Preferably, heating in a patient is effected only to generate a bulk temperature at the body part below a detrimental temperature, e.g. at maximum 50 °C, at maximum 48 °C or at maximum 45 or at maximum 42 °C, depending on the volume affected by heating and depending on the concentration of compounds per volume.

In the table below, exemplary preferred linkers are shown, extending from the carbamate-protected amino group to the carbonyl, for binding a pharmaceutical agent to the carbonyl, and for binding of a nanoparticle to the carbamate-protected amino group, preferably for binding an elongation portion to the carbamate-protected amino group, which elongation portion is bound to a nanoparticle at its end opposite to the carbamate-protected amino group.

Further, exemplary elongation portions are shown. In the elongation portion, a 1,2,3-triazole group can be contained that was produced by a click-reaction of a terminal azide group on one sub-section and a terminal alkinyl group on another sub-section of the elongating portion. A further exemplary reaction for producing an elongating portion is the oxime click reaction.

The linker extending from the Boc-protected amino group to the carbonyl group that forms the second end of the linker, with a linear hydrocarbon chain of 2 to 5 carbon atoms, preferably 2 to 4 or to 3 carbon atoms, arranged between the Boc-protected amino group and the carbonyl group, may be a 2-amino acetic carbonyl moiety, a 3-amino n-propionic carbonyl moiety, a 4-amino n-butanoic carbonyl moiety, a 5-amino pentanoic carbonyl moiety, one valence of the amino group being protected by a Boc group and one valence of the amino group being coupled to an elongating portion that is bound to the nano-particle, directly or with at least one intermediate group, the carbonyl group at the second end of the linker forming an ester bond with a hydroxylated carbon atom of the pharmaceutical agent or forming an amide bond with an amine group of the pharmaceutical agent.

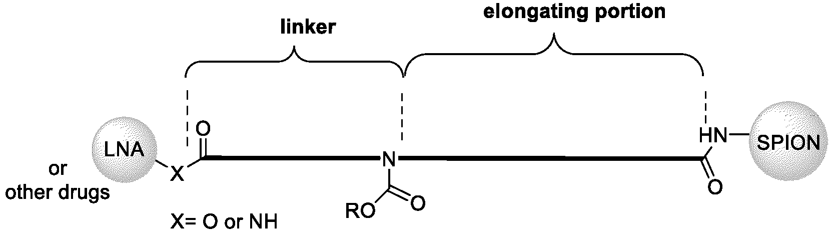

### A. Exemplary, preferred linkers

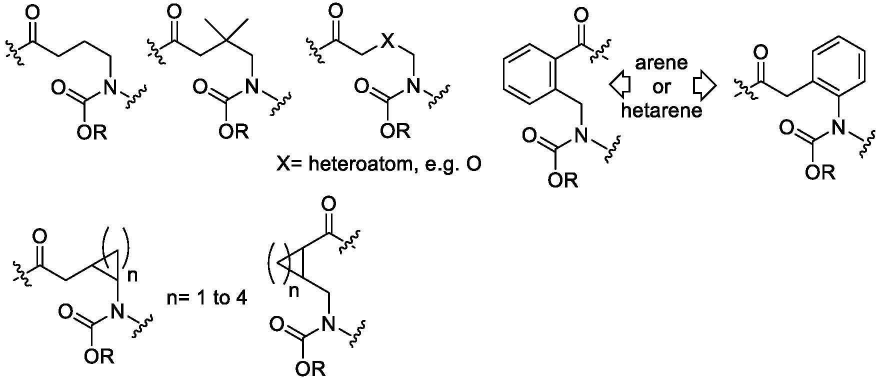

### B. Exemplary, elongation portions

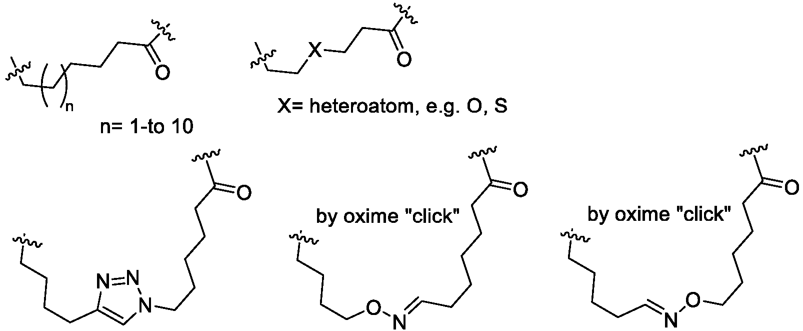

The compound of the invention has the advantage of being stable at body temperature, e.g. at 37 to 40 °C, e.g. up to 39 °C or up to 38°C, and of releasing the pharmaceutical agent from the second end of the linker upon heating. Heating of the compound induces a thermal cleavage of the Boc-protecting group and liberation of a secondary amine which undergoes an intramolecular cyclisation by nucleophilic addition to the carbonyl group arranged at the second end of the linker and bound to the pharmaceutical agent, generating a lactam, preferably a 5-membered or a 6-membered lactam ring, resulting in breaking of the bond between the carbonyl group and the amine group or hydroxyl group of the pharmaceutical agent. As the carbonyl group forms a lactam being part of the linker, the pharmaceutical agent upon heating is released with its original hydroxyl group or its original amine group.

The compound of the invention has the advantage that upon release of the pharmaceutical agent, this pharmaceutical agent is not held back by adhesion to the nano-particle. The release of the pharmaceutical agent without subsequently adhering to the nano-particle or to a coating of the nano-particle is currently believed to be due to the length of the linker which spaces the pharmaceutical agent from the nano-particle or its coating, e.g. in contrast to adherence of pharmaceutical agents to a polymer by surface charges. Generally, the invention also provides a method for medical treatment by administration of the compound to a patient and providing for heating of the compound at a body part for releasing the pharmaceutical agent from the compound.

The pharmaceutical agent that is bound to the carbonyl group of the linker can e.g. be an anti-fibrotic agent, anti-tumor agent, an antibiotic, an antifungal, an antiviral or any other antiinfective, a regeneration- or reparation-inducing agent or in general any agent restoring organ function. Other examples are an anti-fibrotic agent, an anti-inflammatory agent, an immunomodulatory agent, an anti-parasitic agent, a neuroprotective agent, a cardioprotective agent, a metabolic disease modulator (e.g., for diabetes, obesity), an angiogenesis inhibitor or promoter, an antioxidant therapeutic, a pro-apoptotic agent, a gene therapy vector, an epigenetic modulator (e.g., histone deacetylase inhibitors), a stem cell modulator, an anti-autoimmune agent, a radioprotective agent, a chemosensitizer or radiosensitizer, a cell signaling modulator (e.g., kinase inhibitors, receptor agonists/antagonists), a protein degradation inducer (PROTACs, molecular glues, etc.), a hormonal therapy modulator (e.g., estrogen receptor modulators), a blood-brain barrier permeability enhancer or restrictor, a mitochondrial function regulator, a microbiome modulator, a vaccine adjuvant a wound healing accelerator, a senolytic agent (for anti-aging therapies), a stem cell mobilizer, a RNA-modifying agent (e.g., siRNA, mRNA, ASOs), a targeted protein stabilization (molecular chaperones, proteostasis regulators), a tissue regeneration enhancer, a neuroinflammatory suppressor, a lipid metabolism regulator, a synthetic lethality agent (for cancer treatment), an ion channel modulator (e.g., for neurological and cardiac disorders), a metalloproteinase inhibitor (for fibrosis and cancer), a cell adhesion modulator, at toxin neutralizer, an oxygen scavenger or nitric oxide donor (for ischemic diseases).

Preferably, the pharmaceutical agent is a nucleic acid construct, more preferably a locked nucleic acid (LNA) construct, e.g. a phosphothioate-modified RNA, the nucleic acid construct e.g. having a base sequence of an antisense oligonucleotide ((ASO), e.g. an anti-miR).

A preferred nucleic acid construct is an inhibitor of the pro-fibrotic miR21, which inhibitory RNA herein is exemplified by SEQ ID NO: 1 (TCAGTCTGATAAGCT), e.g. with thiophosphate groups in the ribose-phosphate backbone instead of phosphates, e.g. thiophosphates in at least every second ribose moiety, e.g. 5'T*C*A*G*T*C*T*G*A*T*A*A*G*C*T-3' (SEQ ID NO: 1) wherein * designates a thiophosphate bond between ribose moieties linked to a nucleobase, preferably in the form of LNA, termed LNA-21. Generally, a pharmaceutical agent can be an antisense oligonucleotide ((ASO), e.g. anti-miR), e.g. consisting of 10 to 24 nucleotides. Preferably, an ASO is a locked RNA, in the field also referred to as LNA, e.g. having 4'-2'-O-Me-bridged ribose moieties.

For release of the pharmaceutical agent, the compound is heated, e.g. to 85 to 95 °C. For release of the pharmaceutical agent from the compound, heating to a temperature for breaking the bond between the carbonyl group and an amine group or a hydroxyl group of the pharmaceutical agent is sufficient to occur in the immediate vicinity of the nano-particle. The nano-particle preferably is super-paramagnetic such that heating can be effected by application of alternating magnetic fields, e.g. at a frequency of 300 to 400 kHz. Super-paramagnetic nano-particles can e.g. consist of Fe₃O₄, optionally with coating having a functional group that is reactive with the first end of the linker. An exemplary coating is fuctionalized dextran, e.g. bearing amino groups and/or azide groups as functional groups for forming a bond with the first end of the linker.

The compound of the invention is especially suitable for treatment of a part of a patient body by localised release of the pharmaceutic agent, the treatment comprising administration of the compound, e.g. systemic administration, i.e. injection into the blood circulation, followed by application of alternating magnetic fields to a part of a patient body, e.g. to an organ, for inducing release of the pharmaceutic agent in this part. Preferably, the compound is for use in the treatment of a heart, comprising systemic administration of the compound, followed by application of alternating magnetic fields to the heart for release of the pharmaceutic agent within the heart. Preferably in this embodiment, the pharmaceutic agent is an inhibitor for a miR suitable for use in the treatment of a fibrosis of an organ, e.g. fibrotic lung, fibrotic liver, fibrotic kidney, or fibrotic heart disease. For use in the treatment of fibrotic heart disease, the pharmaceutical agent is e.g. a siRNA for inhibiting miR21, especially for use in the treatment of heart infarction and cardiac remodeling.

Optionally, the treatment of a part of a patient body by localised release of the pharmaceutic agent comprises application of permanent, e.g. non-alternating, magnetic fields to the part of a patient body to be treated in order to localize and/or to concentrate compounds of the invention in this part of the body, with concurrent or subsequent by application of alternating magnetic fields to the part of the body in order to induce the release of the pharmaceutic agent from the compound.

The compound of the invention is preferably obtained by process for production, comprising coupling a pharmaceutic agent to a terminal functional group, e.g. an acid group, of a linear hydrocarbon chain of 2 to 5 carbon atoms, preferably 2 to 4 or up to 3 carbon atoms, with a Boc-protected amino group at the opposite end of this hydrocarbon chain forming a linker, and preferably an elongating portion bound to the Boc-protected amino group, wherein a nano-particle is bound to the end elongating portion which is opposite to the Boc-protected amino group.

Generally herein, the secondary amino group of the linker is protected by a Boc-group or by another carbamate, in which the tertiary butyl group of the Boc-group is exchanged for a methyl, ethyl or propyl, or a longer, e.g. a C1 to C6, linear or branched alkyl, or for a benzyl substituent which may further be substituted in the benzene ring, preferentially by a methoxy group. Accordingly, the Boc-protected amino group of the linker also represents a carbamate-protected amino group of the linker. Herein, the Boc-protected amino group is preferred and includes a carbamate-protected amino group, wherein the carbamate is substituted by at least one or two substituents selected from methyl, ethyl or propyl, or a longer, e.g. a C1 to C6, linear or branched alkyl, or for a benzyl substituent which may further be substituted in the benzene ring, preferentially by a methoxy group.

A pharmaceutical agent is coupled via an amino group or via a hydroxyl group to the terminal functional group of the linker, which in case of the terminal functional group being an acid group generating a carbonyl in an amide bond or respectively in an ester bond between the pharmaceutic agent and the linear hydrocarbon chain of the second section. The elongating portion is preferably coupled to the carbamate-protected, e.g. Boc-protected amino group prior to forming the bond of the terminal functional group of the linker to the amine group or hydroxyl group of the pharmaceutical agent. The elongating portion may be bound to the nano-particle before or after coupling the linker to the pharmaceutical agent. The elongating portion may be generated by coupling at least two sub-sections thereof, e.g. by a click-reaction, e.g. by reacting a terminal azide group on one sub-section and a terminal alkinyl group on another sub-section. Therein, one of the sub-sections of the elongating portion may be bound to the carbamate-protected amino group prior to coupling this subsection with another subsection, which may optionally be coupled at its opposite end to the nano-particle or its coating prior to or subsequent to the coupling of the at least two subsections. Alternatively, the elongating portion comprises or consists of a linear polymer, e.g. of the same or a combination of structural elements, e.g. a poly oxyethylene chain (PEG), a fatty acid ester, a dextran, or a fusion of at least two of these.

The invention is now described by way of examples with reference to the figures, which show in
- Fig. 1 a schematic presentation of a synthesis of an exemplary compound of the invention,
- Fig. 2 a schematic presentation of release of a pharmaceutical agent (LNA or other drug) from an exemplary compound of the invention,
- Fig. 3A-H analytical results after administration of compound 80 and after application of alternating magnetic fields (12.5A current, 397 Hz) for plasma markers of liver and kidney function and inflammation, A) for glutamate-pyruvate-transaminase (GPT/ALT), B) glutamate-oxalacetate-transaminase (GOT/AST), C) albumin, D) IL-6, E) bilirubin, F) creatinine, G) urea, and H) temperature profile of different doses of the nanoparticle
- Fig. 4A analytical results for reduction of miR21 levels, from left to right, of PBS control, pure LNA21 (LNA-21), pure dextran-coated super-paramagnetic nano-particles (SPIONs), compound 80 (LNA-21^ SPION),
- Fig. 4 B-E analytical results for plasma markers of liver and kidney function glutamate-oxalacetate-transaminase (AST), glutamate-pyruvate-transaminase (ALT), creatinine, and for urea,
- Fig. 5A -D analytical results, in each case from left to right, of PBS control, pure LNA21 (LNA-21), compound 80 (LNA-21^ SPION), compound 80 with application of alternating magnetic fields (LNA-21^ SPION + AMF), compound 80 with concurrent application of permanent magnet and alternating magnetic fields to the heart region (LNA-21^ SPION + Magnet/ + AMF).

### Example 1: Synthesis of compound with LNA21 as pharmaceutical agent linked to super-paramagnetic nano-particles

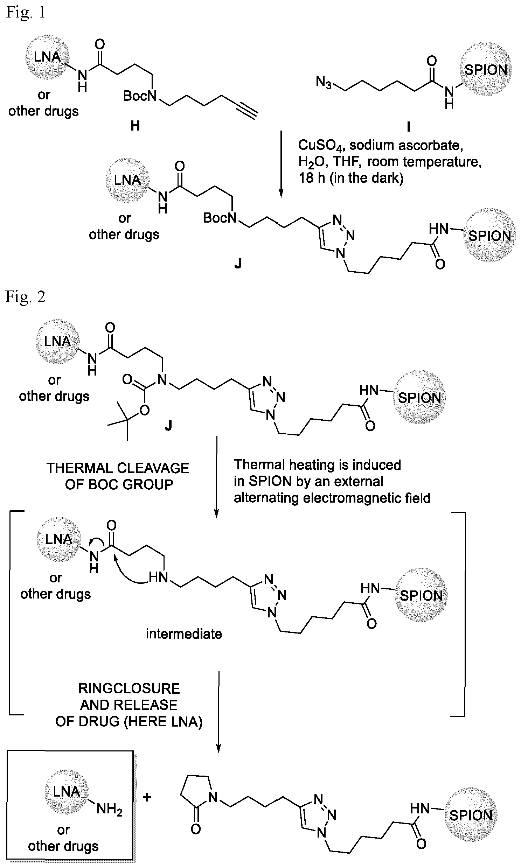

As an exemplary pharmaceutical agent, LNA21 having the nucleotide sequence 5'T*C*A*G*T*C*T*G*A*T*A*A*G*C*T-3' (SEQ ID NO: 1), which is a phosphothioate-modified nucleic acid construct, with * indicating the phosphothioate linkages, of an antisense oligonucleotide specific for the pro-fibrotic microRNA (miR) miR21 was connected to a super-paramagnetic nano-particle by a linker as schematically depicted in Fig. 1. The pharmaceutical agent (LNA) had an amino group as a reactive group terminally arranged on a hexyl group. The linker was composed of a 4-amino butyric carbonyl bound to the reactive amino group of the pharmaceutical agent (LNA), and for generating an elongation portion, one valency of the amino group of the amino butyric carbonyl being protected by Boc, and the other valency of this amino group being bound to an alkinyl group of 71 forming a sub-section of the elongating portion for coupling by a click-reaction to the terminal azide group of an alkyl chain of 74 coupled to a super-paramagnetic nano-particle (SPION) via an amide bond. Therein, the amino group of 74 participating in the amide bond was present on a dextran coating of the nano-particle SPION. The click-reaction between the alkinyl group of 71 and the azide group of 74 yielded the compound 80 of the invention.

### Example 2: Synthesis of compound with LNA21 as pharmaceutical agent linked to super-paramagnetic nano-particles

As an exemplary pharmaceutical agent, LNA21 having the nucleotide sequence 5'T*C*A*G*T*C*T*G*A*T*A*A*G*C*T-3' (SEQ ID NO: 1), a phosphothioate-modified nucleic acid construct of an antisense oligonucleotide specific for the pro-fibrotic microRNA (miR) miR21 was connected to a super-paramagnetic nano-particle by a linker as schematically depicted in Fig. 1. The pharmaceutical agent, herein exemplified by the LNA21, had an amino group as a reactive group terminally arranged on a hexyl group. The linker was composed of 4-amino butyric carbonyl bound to the reactive amino group of the pharmaceutical agent (LNA), one valency of the amino group of the amino butyric carbonyl being protected by Boc, and for generating an elongation portion, the other valency of this amino group being bound to an alkinyl group of compound **H** forming a sub-section of the elongating portion for coupling by a click-reaction to the terminal azide group of an alkyl chain of compound **I** coupled to a super-paramagnetic nano-particle (SPION) via an amide bond. Therein, the amino group of compound **I** participating in the amide bond was present on a dextran coating of the nano-particle SPION. The click-reaction between the alkinyl group of compound **H** and the azide group of compound **I** yielded the compound **K** of the invention.

In detail, aldehyde **B** synthesis was carried out as follows:

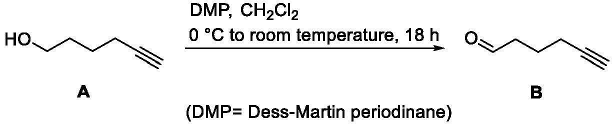

The Dess-Martin-periodinane (2.16 g, 5.10 mmol, 1.00 eq.) was placed in dichloromethane (27.6 mL) and cooled to 0 °C. 5-Hexin-1-ol **(A)** (0.50 g, 5.10 mmol, 1.00 eq.) was slowly added dropwise into the Dess-Martin-periodinane solution and warmed to room temperature (RT) after 5 min. After 18 h, the reaction mixture was filtered through Celite^{™} (ethyl acetate) and the solvent was removed under reduced pressure. After purification by column chromatography (pentane:diethyl ether 6:1), aldehyde compound **B** (0.48 g, 5.00 mmol, 98 %) was obtained as a colourless oil and was directly employed in the reaction.
Rf = 0.28 (pentane:diethyl ether 10:1);
ESI-HRMS: *m*/*z* calculated for C₆H₈ONa [M+Na]⁺: 119.1188, found: 119.1197.

In detail, ester **D** synthesis was carried out as follows: 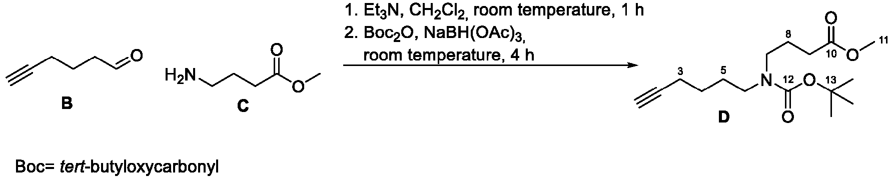 4-Aminobutyric acid methyl ester C (585 mg, 5.00 mmol, 1.00 eq.) and triethylamine (1.7 mL, 12.50 mmol, 2.50 eq.) were added to a solution of aldehyde **B** (0.48 g, 5.00 mmol, 1.00 eq.) in dichloromethane (50 mL). The reaction mixture was stirred for 2 hours before *di-tert-*butyl dicarbonate (1.28 mL, 6.00 mmol, 1.20 eq.) and sodium triacetoxy-borohydride (2.65 g, 12.50 mmol, 2.50 eq.) were added. After 18 hours, the reaction was terminated by adding a saturated sodium hydrogen carbonate solution (50 mL). After phase separation, the aqueous phase was extracted with dichloromethane (3 x 50 mL). The combined organic phases were dried over Na₂SO₄, filtered and the solvent removed under reduced pressure. After purification by column chromatography (petroleum ether:ethyl acetate 10:1), ester **D** (668 mg, 2.25 mmol, 45 %) was obtained as a colourless oil.
***R*_{f}** = 0.33 (petroleum ether/:ethyl acetate 6:1);
**¹H-NMR (400 MHz, CDCl₃)** *δ* = 3.67 (s, 3H, 11-H), 3.19-3.16 (m, 4H, *6-H, 7-H),* 2.31 (t, *J= 7.39* Hz, 2H, 9-H), 2.23-2.19 (m, 2H, 3-H), 1.94 (s, 1H, 1-H), 1.87-1.80 (m 2H, 8-H), 1.66-1.59 (m, 2H, 5-H), 1.53-1.48 (m, 2H, 4-H), 1.44 (s, 9H, *14-H, 15-H,* 16-*H*) ppm; **¹³C-NMR** (100 **MHz, CDCl₃)** *δ* = 173.8 (C10), 155.7 (C12), 84.3 (C2), 79.5 (C13), 68.7 (C1), 51.7 (C11), 46.6 (C7), 46.3 (C6), 31.4 (C9), 28.6 (C14, C15, C16), 27.6 (C5), 25.80 (C4), 23.9 (C8) 18.3 (C3) ppm;
**ESI-HRMS:** *m*/*z* calculated for C₁₆H₂₇NO₄ [M+Na]⁺: 320.1838, found: 320.1831.

In detail, acid **E** synthesis was carried out as follows:

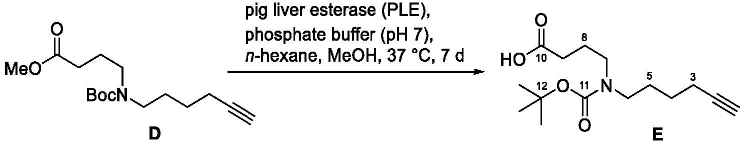

Ester **D** (300 mg, 1.01 mmol, 1.00 eq.) was dissolved in hexane (5.05 mL) and methanol (0.51 mL) before a phosphate buffer solution (pH = 7.4, 50.5 mL) and pig liver esterase (PLE) (48 mg, 720 U) were added. The reaction mixture was warmed up to 37 °C and gently stirred for seven days. Then, the pH was adjusted to 6 by adding hydrochloric acid (6 M) and the reaction mixture was extracted with ethyl acetate (3 x 60 mL). The combined organic phases were washed with an aqueous sodium hydrogen carbonate solution (5 %, 3 x 200 mL), dried over Na₂SO₄, filtered and the solvent removed under reduced pressure. Acid **E** (252 mg, 0.89 mmol, 88 %) was collected as a colourless oil.
***R*_{f}** *=* 0.64 (petroleum ether:ethyl acetate 1:1);
**¹H-NMR (400 MHz, CDCl₃)** *δ* = 3.26-3.18 (m, 4H, *6-H, 7-H),* 2.35 (t, *J =* 7.30 Hz, 2H, 9-H), 2.23-2.20 (m, 2H, 3-H), 1.95 (s, 1H, 1-H), 1.86-1.84 (m, 2H, 8-H) 1.66-1.61 (m, 2H, 5-H) 1.54-1.48 (m, 2H, 4-H), 1.45 (s, 9H, 13-*H,* 14*-H,* 15-*H*) ppm;
**¹³C-NMR** (100 **MHz, CDCl₃)** *δ* = 178.7 (C10), 156.0 (C11), 53.7 (C2), 51.9 (C12), 51.4 (C1), 33.9 (C7), 33.7 (C6), 31.4 (C9), 28.8 (C5), 28.7 (C13, C14, C15), 26.4 (C4), 24.4 (C8), 24.0 (C3) ppm;
**ESI-HRMS:** *m*/*z* calculated for C₁₅H₂₄NO₄ [M-H]: 282.1705, found: 282.1701.

In detail, azide **G** synthesis was carried out as follows: 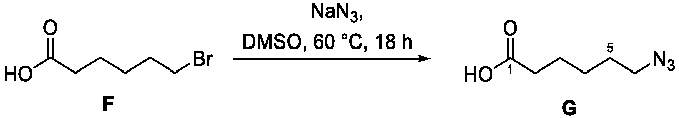 6-Bromohexanoic acid **F** (1.00 g, 5.13 mmol, 1.0 eq.) was dissolved in DMSO (10.3 mL) at room temperature. Sodium azide (1.67 g, 25.63 mmol, 5.0 eq.) was added slowly and the reaction mixture was heated to 60 °C. After 18 hours, the reaction was terminated by adding dist. water (10 mL). After addition of dichloromethane (10 mL), the phases were separated and the aqueous phase was extracted with dichloromethane (3 x 15 mL). The combined organic phases were dried over Na₂SO₄, filtered and the solvent removed under reduced pressure. After purification by column chromatography (CH₂Cl₂:MeOH 20:1), azide **F** (0.66 g, 4.20 mmol, 82 %) was collected as a colourless oil.
***R*_{f}** *=* 0.50 (CH₂Cl₂:MeOH 6:1);
**¹H-NMR (400 MHz, CDCl₃)** *δ* = 11.37 (bs, 1H, 1-OH), 3.27 (t, *J =* 6.84 Hz, 2H, 2-H), 2.36 (t, *J =* 7.40 Hz, 2H, 6-H), 1.64 (m, 4H, 4*-H,* 5-H), 1.42 (m, 2H, 3-*H*) ppm;
**¹³C-NMR (100 MHz, CDCl₃)** *δ* = 180.2 (C1), 51.3 (C6), 33.9 (C2), 28.6 (C5), 26.2 (C4), 24.2 (C3) ppm;
**ESI-HRMS:** *m*/*z* calculated for C₆H₁₀N₃O₂ [M+H]⁺: 156.0773, found: 156.0769.

An alkyne-modified linker bound to LNA **I** was prepared as follows: 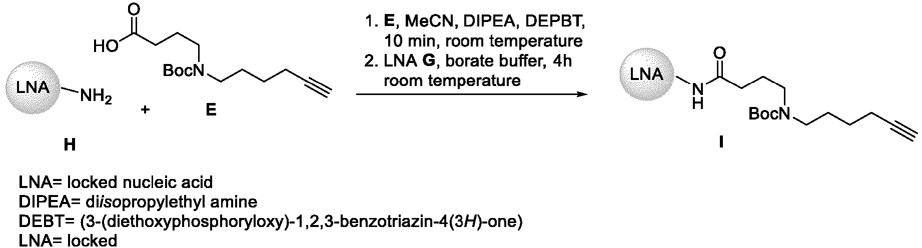 Acid **E** (200 mM in acetonitrile, 5 µL), DIPEA (200 mM in acetonitrile, 5 µL) and DEPBT (200 mM in acetonitrile, 5 µL) were mixed in a thermal shaker for 10 minutes at room temperature. Amino-modified LNA **H** and borate buffer (0.5 M, pH = 9.5, 8 µL), LNA-21 (10 nmol in 5 µL H₂O) and dist. H₂O (5 µL) were added and the reaction mixture was left at room temperature for 4 hours without mixing. Addition of ethanol (91 µL) and an aqueous sodium chloride solution (5 M, 2 µL) terminated the reaction and the solution was shaken overnight at -20 °C. After centrifugation (14.8 rpm, 4 °C, 30 min), the supernatant was decanted, the pellet, that contained LNA conjugate **I,** was taken up in ethanol (100 µL) and centrifuged again (14.8 rpm, 4 °C, 15 min). Decantation, collection in ethanol (100 µL) and centrifugation (14.8 rpm, 4 °C, 15 min) was repeated once more before the pellet I was dried in vacuo.

Azide-functionalized super-paramagnetic iron oxide nanoparticles (SPION) **J,** Fe₃O₄, ca. 130 nm diameter, dextran coated with NH₂ groups, (Perimag, available from micromod Partikeltechnologie, Rostock, Germany) were prepared as follows:

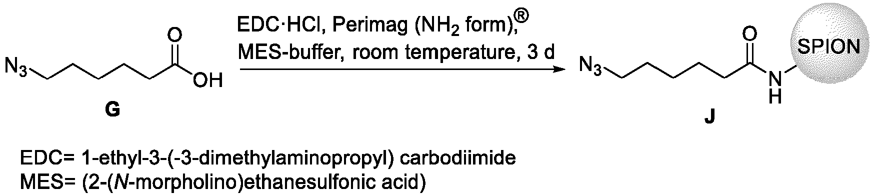

Azide **G** (1.2 mg, 7.64 µmol) and EDC-HCl (1.5 mg, 7.82 µmol) were dissolved in an aqueous MES buffer (0.5 M, 0.3 mL) in a thermal shaker. After 10 minutes at 50 °C superparamagnetic iron oxide nanoparticles (SPION) coated with amino-functionalized Dextran (5 to 10 nmol amino groups per 1 mg iron (Perimag^{®}-NH₂, 10 mg iron/mL, 1 mL)) was added and the reaction mixture was kept in the thermal shaker for three days at room temperature. The solution was then transferred to a dialysis tube and dialysed against deionised water for a period of five days. Water was exchanged three times a day. After completion of dialysis, SPION conjugate **J** (10 mg iron/5.5 mL H₂O) was transferred to Sarstedt tubes and stored at 4 °C.

Click coupling of alkyne-modified LNA **I** with azide-modified SPION **J** and preparation of conjugate **K:**

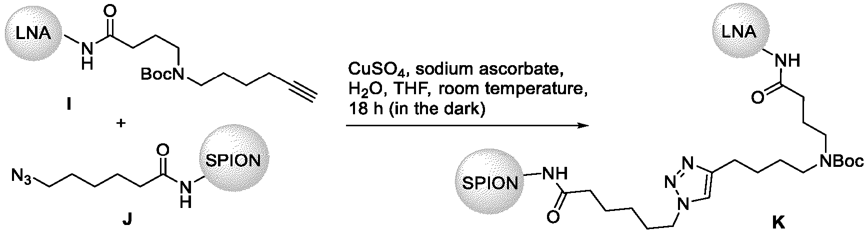

Azide-modified LNA **I** (25 nmol) was dissolved in H₂O (75 µL) and modified SPION **J** (10 mg iron/5.5 mL H₂O). THF (2 µL), CuSO₄ (cat.) and sodium ascorbate (cat.) were added successively and the reaction mixture was gently stirred for 18 hours in the absence of light. The solution was then transferred to a dialysis tube and dialysed against deionised water for four days. Water was changed three times a day. After completion of dialysis, SPION-LNA LNA **K** (10 mg iron/7.5 mL H₂O) was transferred to plastic tubes (Sarstedt) and stored at 4 °C.

Release of LNA **H** from conjugate **K** in the presence of an external oscillating electromagnetic field:

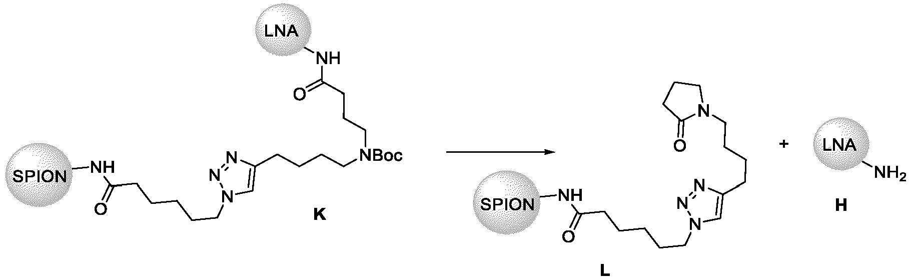

For release of LNA **H,** thermal heating of the compound to 85 to 95 °C as measured in the suspension, was found to be very efficient leading to ca. 50% to 75% of LNA **H** in solution after 5 to 10 min and 81% ca. 30 to 40 min. As oscillating electromagnetic field primarily act on the superparamagnetic nanoparticles (SPIONs), heat is generated inside the nanoparticle and its direct vicinity. Consequently, the linker and the pharmaceutical agent are also exposed to this heat. The advantage of this kind of thermal release system lies in the fact that this invention is well suited for release of pharmaceutic agents by use of external oscillating electromagnetic fields, thereby avoiding heating of the bulk volume including cell tissue.

For these exemplary compounds, susceptibility measurements determined a hydrodynamic diameter of the compound of 170 nm, and after release of the LNA **H** by heating using electromagnetically alternating magnetic fields, the hydrodynamic diameter was determined to be 134 nm. The decrease of the hydrodynamic diameter indicates release of the LNA. Therein, measurement of the hydrodynamic diameter was by dynamic light scattering (DLS). DLS is based on the Brownian motion of dispersed particles and the particle size is calculated from the translational diffusion coefficient of the particles using the Stokes-Einstein equation.

Using the example of a compound according to the invention, Fig. 2 shows the reaction for releasing the active pharmaceutical ingredient, herein exemplified by the LNA, from the conjugate compound, the release being triggered by heating, whereby the Boc group is first cleaved off and the secondary amine released in this way nucleophilically attacks the carbonyl group with the esterified active ingredient, whereby the active ingredient is released with the formation of a lactam from the linker.

### Example 3: Cytotoxicity measurements

For determination of cytotoxicity, murine cardiac fibroblasts, cardiomyocytes, renal cells, and liver cells in cell culture were contacted with Linker A (compound 55), Linker B (compound 56), LNA-Linker A portion conjugate (compound 71), Linker B coupled to SPION (compound 74), the complete compound comprising LNA21-linker-SPION (compound 80), or compound 80 plus application of alternating magnetic fields (326 to 397 kHz, 6,25 to 25 A applied to coil of four windings, internal diameter 5.5 cm or coil of 6 windings, internal diameter 3.0 cm), each at 500 nM, 100 nM, 50 nM or 10 nM final concentration. Cells were seeded to 500 000 in a cell culture vessel (T150 from TPP) with fibroblast growth medium 3 (Promocell) with added 10 vol.-% FBS (Gibco), 1 % P/S (10 mL, Gibco) and incubated at 37 °C, 5 % CO₂ atmosphere. After 4 days, the medium was changed, cells were sub-cultured after 7 days by washing in PBS and trypsinization. For these assays, cells were used at passages 4 to 8.

Measurement of lactate dehydrogenase (LDH) used the Cyto Tox 96^{®} non-radioactive cytotoxcity reagent (Promega), measuring luminescence (Synergy HT Reader). Cardiac fibroblasts, cardiomyocytes, renal cells and liver cells did not show any statistically significant changes over untreated control cells, measurement of caspase 3/7 (Caspase-Glo^{®} 3/7 assay by Promega) for cardiac fibroblasts did not show any statistically significant (Student's t-test) changes over untreated control cells.

As a result, the compound of the invention, without and with application of alternating magnetic fields, as well as precursors used for its synthesis was found to be not cytotoxic

### Example 4: In-vivo release of pharmaceutical agent from a compound of the invention for treatment of heart disease

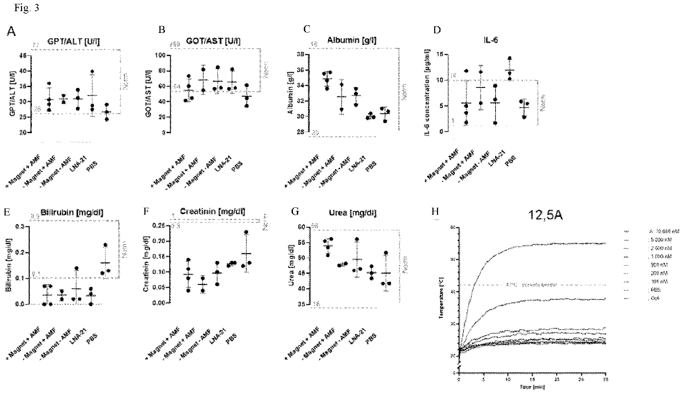

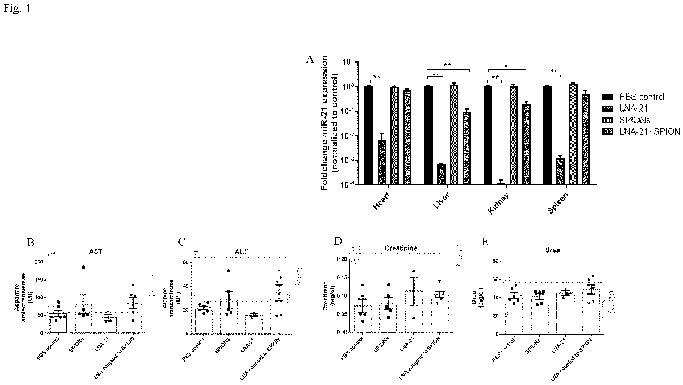

Initially, 7 C57BL/6N mice were intravenously injected in the highest tested dose of compounds of the invention according to Example 1, the compound 80 consisting of the LNA21 as the pharmaceutical agent, the linker and elongating portion according to Example 1 and the dextran-coated super-paramagnetic nano-particles, showing that the compound is safe and not toxic. C57BL/6N mice were intravenously injected with the compound 80, or as a control injected with dextran-coated super-paramagnetic nano-particles only, or pure LNA-21 only, or PBS. Organs were harvested after 7 days. Six out of seven mice which were injected with the compound 80 and five out of seven mice injected with pure super-paramagnetic nano-particles survived, indicating that the compound of the invention even in the highest dose of possible iron content was tolerated by these mice. Parameters of kidney and liver damage were analysed, results are depicted in Fig. 3 as measured in plasma samples showing that there were no signs of organ damage. Also, histological examinations revealed non-toxic effects in all organs, strengthening the finding that the compound of the invention is safe for in vivo use. Fig. 3H shows bulk temperature of compound 80 of the invention at a concentration of 5 µM suspended in water, corresponding to the blood concentration of the compound reached in these injections under an alternating magnetic field as used in this example of 25,5 mT for 30 min, generated by a current of 12.5 A at 397 Hz by 6 windings. Fig. 4A shows the gene expression for miR21 levels in the different organs indicated, in each case from left to right for PBS as a negative control, for pure LNA21 (LNA-21) showing drastic reduction of levels of miR21, for pure dextran-coated super-paramagnetic nano-particles (SPIONs) showing no effect on miR21 levels, and for the compound 80 of the invention, which in the absence of alternating magnetic fields showed only little effect on reducing miR21 levels. In summary, quantification of miR21 levels in all organs showed that the systemic injection of LNA21 led to a complete suppression of miR21 in almost all organs, while compound 80 of the invention showed only little effect for suppressing miR21 in heart and spleen, but a more pronounced effect in liver and kidney.

Fig. 4A shows the analytical results for miR21 levels in the different organs indicated, in each case from left to right for PBS as a negative control, for pure LNA21 (LNA-21) showing drastic reduction of levels of miR21, for pure dextran-coated super-paramagnetic nano-particles (SPIONs) showing no effect on miR21 levels, and for the compound 80 of the invention, which in the absence of alternating magnetic fields showed only little effect on reducing miR21 levels. In summary, quantification of miR21 levels in all organs showed that the systemic injection of LNA21 led to a complete suppression of miR21 in almost all organs, while compound 80 of the invention showed only little effect for suppressing miR21 in heart and spleen, but a more pronounced effect in liver and kidney. Fig. 4 B-E show analytical results for plasma markers of liver and kidney function, indicating that the plasma markers analysed were not affect to a level outside the norm (Norm, range indicated as lower and upper level). The low creatinine levels were determined also in the controls (PBS control, SPIONs, LNA-21), indicating that the low creatinine levels were not caused by the compound of the invention (LNA coupled to SPION).

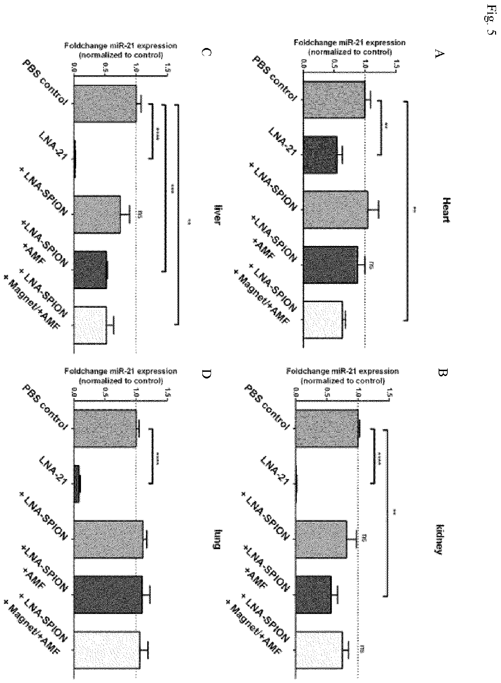

Fig. 5 shows analytical results, in Fig. 5A for heart, in Fig. 5B for kidney, in Fig. 5C for liver, and in Fig. 5D for lung. The release of the LNA21 from the compound 80 of the invention and the effect of LNA21 on miR21 levels was analysed in different groups of mice: 1) injection of the compound 80 of the invention but without application of altering magnetic fields, therefore no release of LNA21 expected, 2) injection of the compound 80 of the invention plus application of altering magnetic fields, therefore release LNA21 is expected, and 3) injection of the compound 80 of the invention in combination with an external permanent magnet placed on the beating heart while injecting compound 80 of the invention, in an attempt to accumulate the compound 80 in the region of the heart with subsequent application of altering magnetic fields in the region of the heart, for expected release of LNA21. 2 days after these treatments, mice were killed and organs were harvested and expression of miR21 was quantified, serving as an indicator for the release and effect of LNA21. It was found that injection of pure LNA21 (second column from left, Fig. 5A-D) resulted in decrease of miR21 in the organs analysed, indicating huge off-target effects in these organs, while in the heart miR21 expression was decreased by only 50%. The combined application of the external permanent magnet and alternating magnetic fields (right column, Fig. 5A-D) showed lower decrease of miR21, indicating a significant reduction in off-target effects, especially in organs such as liver and kidney, whereas the efficiency of decrease of miR21 in the heart remained unaltered compared to administration pure LNA21, indicating that upon application of the alternating magnetic fields (AMF) the compound 80 of the invention released LNA21 (fourth column from left, Fig. 5A-D). The application of the permanent magnetic force in the region of the heart in combination with application of the alternating magnetic fields the compound 80 of the invention released LNA21, resulting in higher efficiency of decrease of miR21 (right column, Fig. 5A-D).

RNA was isolated using the Qiazol reagent (Qiagen) after washing cells in PBS, followed by extraction with chloroform and precipitating RNA from the supernatant with iso-propanol by centrifugation, then washing the pellet with 75% ethanol in water and drying then resuspending the pellet in RNAse-free water. Determination of miRNA levels was by quantitative PCR using the TaqMan MicroRNA Assay (Applied Biosystems), 95°C for 15 min, 45 cycles of 95°C for 15 s and 60°C for 60 s.

IL6 in murine blood plasma was measured using the Mouse IL6 Uncoated ELISA (Invitrogen).

## Claims

1. Compound comprising a nano-particle coupled to a first end of a linker, the opposite second end of the linker terminating in a carbonyl group, which carbonyl group is bound to a pharmaceutical agent, wherein the linker carries the carbonyl group at the terminus of a linear hydrocarbon chain of 2 to 5 carbon atoms bound to a secondary amino group of the linker, the secondary amino group being protected by an alkyl carbamate group.

2. Compound according to claim 1, **characterized in that** the carbonyl group is bound to an amine group or a hydroxyl group of the pharmaceutical agent forming an ester or amide group.

3. Compound according to one of the preceding claims, **characterized in that** the alkyl carbamate group consists of a carbonyl group having a bound oxy-alkyl, the alkyl consisting of C1 to C6, linear or branched alkyl.

4. Compound according to one of the preceding claims, **characterized in that** the nano-particle is a super-paramagnetic iron oxide nano-particle (SPION).

5. Compound according to one of the preceding claims, **characterized in that** the nanoparticle is bound to the carbamate-protected secondary amino group of the linker by an elongating portion.

6. Compound according to one of the preceding claims, **characterized in that** the elongating portion is formed by a coat of intermediate groups attached to the nano-particle, each of which intermediate groups is a hexose, a pentose or a combination of at least two of these, or a fusion of at least two of these.

7. Compound according to one of the preceding claims, **characterized in that** opposite the linear hydrocarbon chain, the carbamate-protected secondary amino group of the linker is bound to the nano-particle by a elongating portion, which elongating portion has a chain length of at least 10 atoms up to 20 atoms.

8. Compound according to one of the preceding claims, **characterized in that** the linker extends from the carbamate-protected amino group to the carbonyl group that forms the second end of the linker, the linker having a linear hydrocarbon chain of 2, 3, 4 or 5 carbon atoms arranged between the carbamate-protected amino group and the carbonyl group, one valence of the carbamate-protected amino group being protected by a carbamate group and one valence of the amino group being coupled to the elongating portion that is bound to the nano-particle.

9. Compound according to one of the preceding claims, **characterized in that** at least one carbon atom of the linker is derivatized by at least one group or two groups which are independently selected from C1- to C6- aliphatic groups, e.g. methyl, di-methyl, ethyl, di-ethyl, halogen, or a combination of two of these.

10. Compound according to one of claims 1 to 8, **characterized in that** at least two carbon atoms of the linker are derivatized by forming part of an ortho-substituted (hetero)arene or of a 1,2-disubstituted aliphatic ring system, which is a 3-, a 4-, a 5- or a 6-membered ring, wherein substituents to the arene or aliphatic ring are independently be selected from hydrogen, C1- to C6 aliphatic groups, and halogen.

11. Compound according to one of the preceding claims, **characterized in that** the linker including its carbonyl group and its amino group has a chain length of 5 or 6 carbon atoms, wherein one of the carbon atoms is optionally replaced by an oxygen or a sulfur atom.

12. Compound according to one of the preceding claims, **characterized in that** the pharmaceutical agent bound to the carbonyl group of the linker can e.g. be an anti-fibrotic agent, anti-tumor agent, an antibiotic, an antifungal, an antiviral or any other antiinfective, a regeneration- or reparation-inducing agent or in general any agent restoring organ function. Other examples are an anti-fibrotic agent, an anti-inflammatory agent, an immunomodulatory agent, an anti-parasitic agent, a neuroprotective agent, a cardioprotective agent, a metabolic disease modulator (e.g., for diabetes, obesity), an angiogenesis inhibitor or promoter, an antioxidant therapeutic, a pro-apoptotic agent, a gene therapy vector, an epigenetic modulator (e.g., histone deacetylase inhibitors), a stem cell modulator, an anti-autoimmune agent, a radioprotective agent, a chemosensitizer or radiosensitizer, a cell signaling modulator (e.g., kinase inhibitors, receptor agonists/antagonists), a protein degradation inducer (PROTACs, molecular glues, etc.), a hormonal therapy modulator (e.g., estrogen receptor modulators), a blood-brain barrier permeability enhancer or restrictor, a mitochondrial function regulator, a microbiome modulator, a vaccine adjuvant a wound healing accelerator, a senolytic agent (for anti-aging therapies), a stem cell mobilizer, a RNA-modifying agent (e.g., siRNA, mRNA, ASOs), a targeted protein stabilization (molecular chaperones, proteostasis regulators), a tissue regeneration enhancer, a neuroinflammatory suppressor, a lipid metabolism regulator, a synthetic

13. Compound according to one of the preceding claims for use in medical treatment, the treatment comprising administration of the compound to a patient and applying energy to a part of a patient body for heating the compound for inducing release of the pharmaceutic agent from the compound.

14. Compound for use in medical treatment according to claim 13, wherein applying energy for heating is by application of alternating magnetic fields to a part of a patient body.

15. Compound for use in medical treatment according to one of claims 13 to 14, wherein applying energy for heating to a part of a patient body results in release of the pharmaceutical agent localised in the part of a patient body.

16. Compound for use in medical treatment according to one of claims 13 to 15, wherein administration of the compound is systemic administration or local injection.

17. Compound for use in medical treatment according to one of claims 13 to 16, wherein the treatment comprises application of permanent magnetic fields to the part of a patient body to be treated in order to localize and/or to concentrate compounds of the invention in this part of the body.
